# EUROPÄISCHE PATENTSCHRIFT

(11) **EP 0 355 598 B1**
(45) Veröffentlichungstag und Bekanntmachung des Hinweises auf die Patenterteilung: **16.06.1993**
(21) Anmeldenummer: 89114787.8
(22) Anmeldetag: 10.08.1989
(51) Int. Cl.: C07C 309/49, C07C 217/22, C07D 267/14, C09B 19/02, C09B 62/503

(54) **Beta-Phenoxyethylamine und deren Verwendung zur Herstellung von Farbstoffen**
Beta-phenoxyethyl amines and their use in the preparation of dyes
Bêta-phénoxyéthylamines et leur utilisation pour la préparation de colorants

(30) Priorität: 23.08.1988 DE 3828493
(43) Veröffentlichungstag der Anmeldung: 28.02.1990
(73) Patentinhaber: BAYER AG, 51368 Leverkusen (DE)
(72) Erfinder: Herd, Karl Josef, Dr., D-5068 Odenthal (DE); Harms, Wolfgang, Dr., D-5068 Odenthal (DE)

(56) Entgegenhaltungen:
- EP-A- 0 245 603
- EP-A- 0 299 328
- DE-A- 1 670 759
- DE-A- 3 635 312
- CHEMICAL ABSTRACTS, Band 71, 15. September 1969, Seite 393, Zusammenfassung Nr. 49919m, Columbus, Ohio, US; U.T. BHALERAO et al.: "Preparation and N.M.R. spectral data of some 1,4-benzoxazepinones"

## Beschreibung

Gegenstand der vorliegenden Erfindung sind ß-Phenoxyethylamine der Struktur 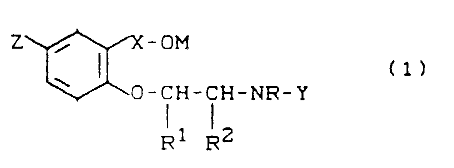
worin
- Z =: NH₂ oder NO₂
- M =: H, Alkali-, Erdalkali- oder Ammoniumkation
- R =: H oder ein gegebenenfalls substituierter (insbesondere durch OH, Cl, Br, CN, CO₂H, CO₂CH₃, CO₂C₂H₅, CONH₂, CON(CH₃)₂, SO₃H, OSO₃H sowie C₁-C₄-Alkoxy) C₁-C₄-Alkyl-, C₂-C₄-Alkenyl- oder C₂-C₄-Alkinyl-Rest oder
ein gegebenenfalls substituierter (insbesondere durch Halogen, C₁-C₄-Alkyl, CO₂H oder SO₃H) Phenyl-oder Benzyl-Rest,
- R¹, R² =: unabhängig voneinander H, C₁-C₄-Alkyl, Phenyl oder Benzyl, wobei R¹-R² auch Bestandteil eines cycloaliphatischen Restes sein können,
- X =: SO₂ oder CO und
- Y =: H oder SO₃M,
wobei
- Y =: SO₃M, wenn X = SO₂ und
- Y =: H, wenn X = CO.

Bevorzugte Verbindungen (1) sind solche mit
R¹ und R² = H und R = C₁-C₄-Alkyl, CH₂CH₂OH, CH₂CO₂H oder CH₂CH₂OSO₃H.

Gegenstand der Erfindung ist auch ein Verfahren zur Herstellung der Phenoxyethylamine (1), dadurch gekennzeichnet, daß man 2-Halogen-5-nitrobenzolsulfonsäureamide bzw. 2-Halogen-5-nitrobenzoesäureamide der Strukturen (2) (aus 2-Halogen-5-nitrobenzolsulfonsäurechlorid bzw. 2-Halogen-5-nitrobenzoesäurechlorid und den entsprechenden Aminen nach bekannten Methoden darstellbar), 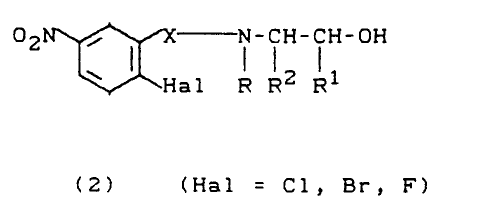
worin
X, R, R¹ und R² die unter Formel (1) genannte Bedeutung zukommt,
bei 80° bis 110°C in Gegenwart von Basen, insbesondere Alkalihydroxiden oder Alkalicarbonaten wie Natronlauge, Kalilauge, Soda, zu 5,1,2- Benzoxathiazepin-1,1-dioxiden (X = SO₂) bzw. 1,4-Benzoxazepin-5-onen (X=CO) der Formel (3) 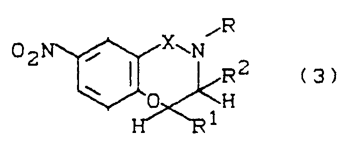
cyclisiert, diese Benzosultame bzw. Benzolactame anschließend katalytisch (z.B. Wasserstoff/Katalysator), mit Metall/Säure (z.B. Eisen/Essigsäure) oder mit anderen für aromatische Nitroverbindungen üblichen Reduktionsmitteln zu Aminoverbindungen der Struktur (4) reduziert 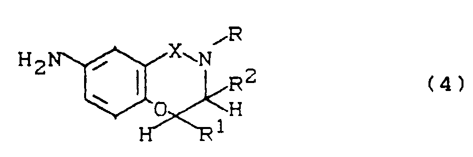
und diese abschließend unter Ringöffnung hydrolysiert. Die Hydrolyse der Benzosultame (X = SO₂) erfolgt dabei bevorzugt in Oleum bei 60 - 120°C und liefert einheitlich die beanspruchten Amidosulfonsäuren der Struktur (5). 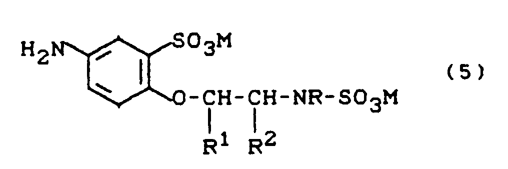
Durch nachfolgende Neutralisation sind die entsprechenden Alkali-, Erdalkali-, bzw. Ammoniumsalze oder Betaine zugänglich.

Die Hydrolyse der Benzolactame (X = CO) erfolgt am geeignetsten mit verdünnten Mineralsäuren, wie verdünnter Salzsäure oder Schwefelsäure bei Reaktionstemperaturen von 80 -110°C und liefert gegebenenfalls nach Neutralisation die beanspruchten carboxysubstituierten ß-Phenoxyethylamine der Struktur (6) oder deren Hydrochloride bzw. Hydrogensulfate. 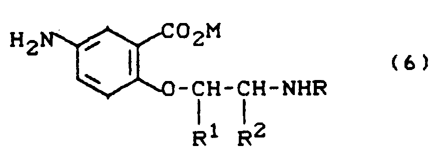
Gegenstand der Erfindung ist auch ein Verfahren zur Herstellung der Phenoxyethylamine der Struktur (1), dadurch gekennzeichnet, daß man Benzosultame bzw. Benzolactame der Struktur (3) zunächst unter Ringöffnung zu Verbindungen der Formel (7) bzw. (8) hydrolysiert, 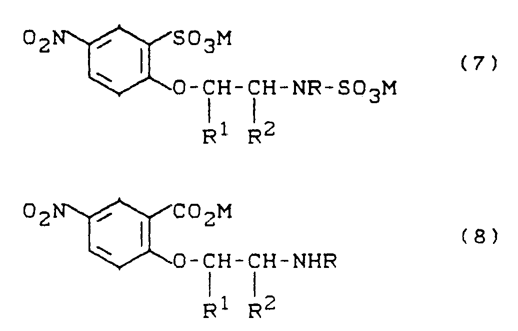
wobei Benzosultame (3, X = SO₂) bevorzugt bei 60 bis 120°C in Oleum zu (7) und Benzolactame (3, X = CO) bevorzugt bei 90 bis 110°C in verdünnten Mineralsäuren hydrolysiert werden, und abschließend die erhaltenen Nitroverbindungen (7) bzw. (8) zu Aminoverbindungen der Struktur (5) bzw. (6) reduziert. Die Reduktion kann katalytisch (z.B. Wasserstoff/Raney-Nickel-Katalysator), mit Metall/Säure oder anderen für aromatische Nitrogruppen gebräuchlichen Reduktionsmitteln durchgeführt werden.

Die Substitutions- bzw. Cyclisierungsreaktion zu Verbindungen der Struktur (3) führt man in Anlehnung an DE-A 1 670 759 oberhalb 80°C, am vorteilhaftesten zwischen 90 - 105°C, gegebenenfalls unter Druck, durch. Als Reaktionsmedium dient in erster Linie Wasser; es kann aber auch ein organisches wassermischbares Lösungsmittel,
z.B. Alkohol, DMF oder Dimethylsulfoxid, oder eine Mischung dieser mit Wasser verwendet werden. Vorzugsweisearbeitet man in Wasser unter Rückflußbedingungen. Die Hilfsbase wird zur Deprotonierung der OH-Funktion in (2) und zum Abfangen des bei der Kondensation freiwerdenden Halogenwasserstoffes H-Hal benötigt. Die Substitutionsreaktion verläuft einheitlich intramolekular.

Als besonders geeignet für die Hydrolyse der Verbindungen (3, X = SO₂) und (4, X = SO₂) erweist sich der Einsatz von 20 %igem Oleum bei 75 - 90°C.

Gegenstand der Erfindung sind auch neue 7-Amino-1,4-benzoxazepin-5-one der Struktur (9), 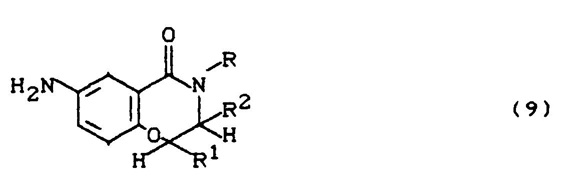
die als Zwischenprodukte (4) mit X = CO im beanspruchten Herstellungsverfahren durchlaufen werden, insbesondere 7-Amino-2,3-dihydro-(5H)-1,4-benzoxazepin-5-on, 7-Amino-4-methyl-2,3-dihydro-(5H)-1,4-benzoxazepin-5-on sowie 7-Amino-4-(2′-hydroxyethyl)-2,3-dihydro-(5H)-1,4-benzoxazepin-5-on.

Ebenso Gegenstand der Erfindung ist auch ein Verfahren zur Herstellung von Zwischenprodukten der Struktur (3) mit X = SO₂ und R = gegebenenfalls substituiertes C₁-C₄-Alkyl, C₂-C₄-Alkenyl oder C₂-C₄-Alkinyl, dadurch gekennzeichnet, daß man Verbindungen der Struktur (3) mit X = SO₂ und R = H unter Basenkatalyse mit Alkylierungsreagenzienwie R-Hal oder Epoxiden, wie z.B. Chloressigsäure oder Ethylenoxid, oder aktivierten Doppelbindungssystemen, wie z.B. Acrylnitril oder Acrylsäureestern, alkyliert. Bevorzugtes Reaktionsmedium ist dabei Wasser, die Reaktionstemperatur liegt im Bereich von 50 - 110°C.
Beansprucht wird auch ein Verfahren zur Herstellung von β-Phenoxyethylaminen der Struktur (10), wie sie z.B. in der DOS 3 635 312 beschrieben sind, 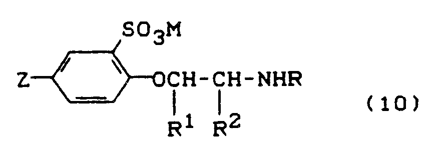
mit Z = NO₂ oder NH₂,
dadurch gekennzeichnet, daß man Amidosulfonsäuren der Strukturen (5) und (7) mit verdünnten Mineralsäuren, insbesondere Salzsäure oder Schwefelsäure unter Rückflußbedingungen oder bei erhöhten Temperaturen unter Druck hydrolysiert.

Beansprucht wird auch die Verwendung der neuen beanspruchten Verbindungen der Strukturen (1), (5), (6), (7), (8) und (9) zur Herstellung von Farbstoffen, speziell der Verbindungen mit Z = NH₂ als Diazokomponenten in der Azofarbstoffchemie bzw. als Kondensationskomponenten für aktivierte Chinone, wie Chloranil, zum Aufbau von Triphendioxazin-Farbstoffen.

Für die Herstellung von Triphendioxazin-Farbstoffen werden Verbindungen der Formel (1) oder (10) mit Z = NH₂ in bekannter Weise mit 1,4-Benzochinonen zu Farbstoffvorstufen der Formel (11), 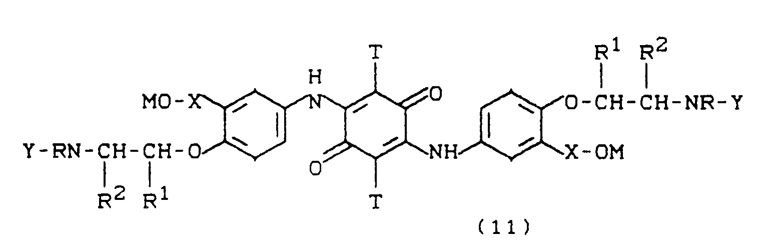
worin
- T =: Cl, Br, CO₂CH₃, OCH₃, 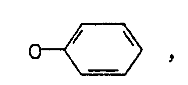
- X =: SO₂ oder CO,
- Y =: H oder SO₃M
und M, R, R¹ und R² die unter Formel (1) genannte Bedeutung zukommt,
kondensiert und anschließend einen Ringschluß zu Triphendioxazinfarbstoffen der Formel 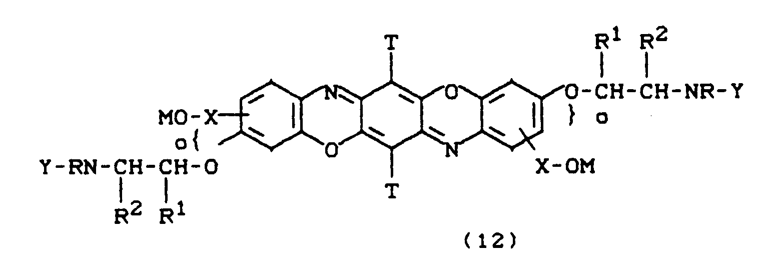
durchführt,
wobei die X-OM-Reste jeweils in einer der ortho-Positionen zum Aminoethoxy-Rest stehen. Der Ringschluß kann beispielsweise nach Methoden, wie sie in den Deutschen Offenlegungsschriften 2 122 262, 2 124 080, 2 302 382, 2 344 781, 2 503 611, 2 823 828 und in der Britischen Patentschrift 2 019 872 beschrieben sind, erfolgen. Farbstoffe der Formel (12) mit Y = SO₃M lassen sich mit verdünnten Mineralsäuren zu solchen mit Y = H hydrolysieren.

Farbbasen der Formel (12) mit Y = H lassen sich auch mit Reaktivkomponenten der Struktur Q-Hal (Q-faserreaktiver Rest, Hal = Cl, Br, F) zu teilweise bekannten faserreaktiven Triphendioxazinfarbstoffen der Formel (12) mit Y = Q, worin Q = faserreaktiver Rest, insbesondere aus der Triazin- bzw. Pyrimidin-Reihe, umsetzen (vg. DE-A 3 635 312).

Typische Farbstoffbeispiele sind: 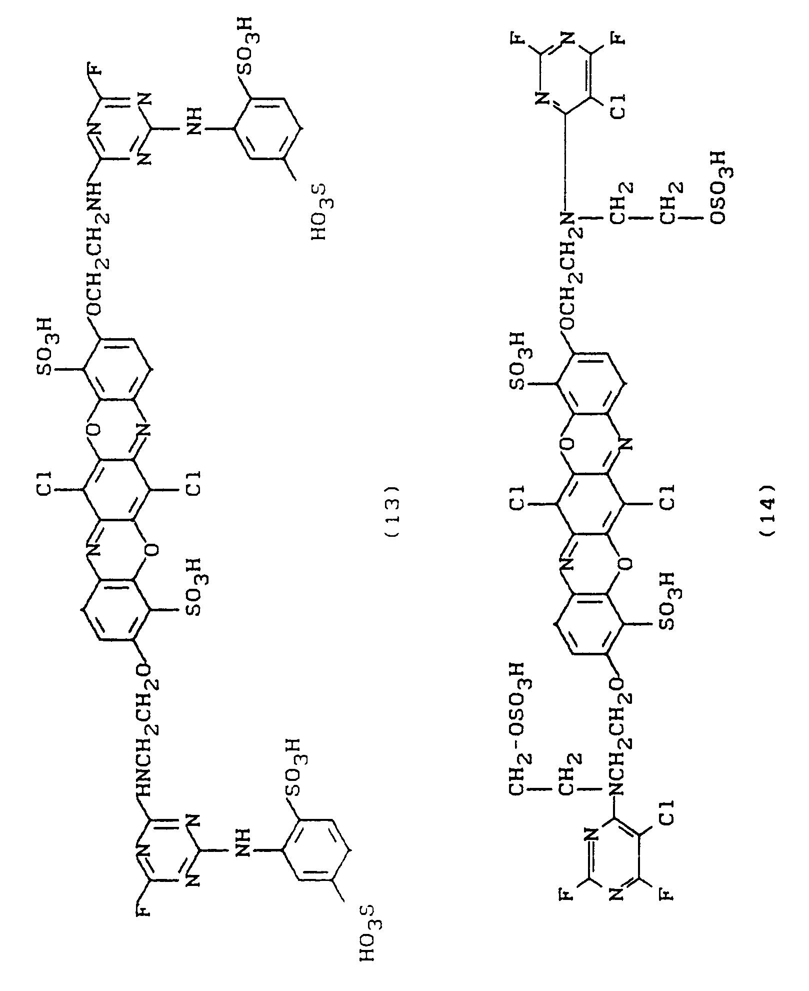 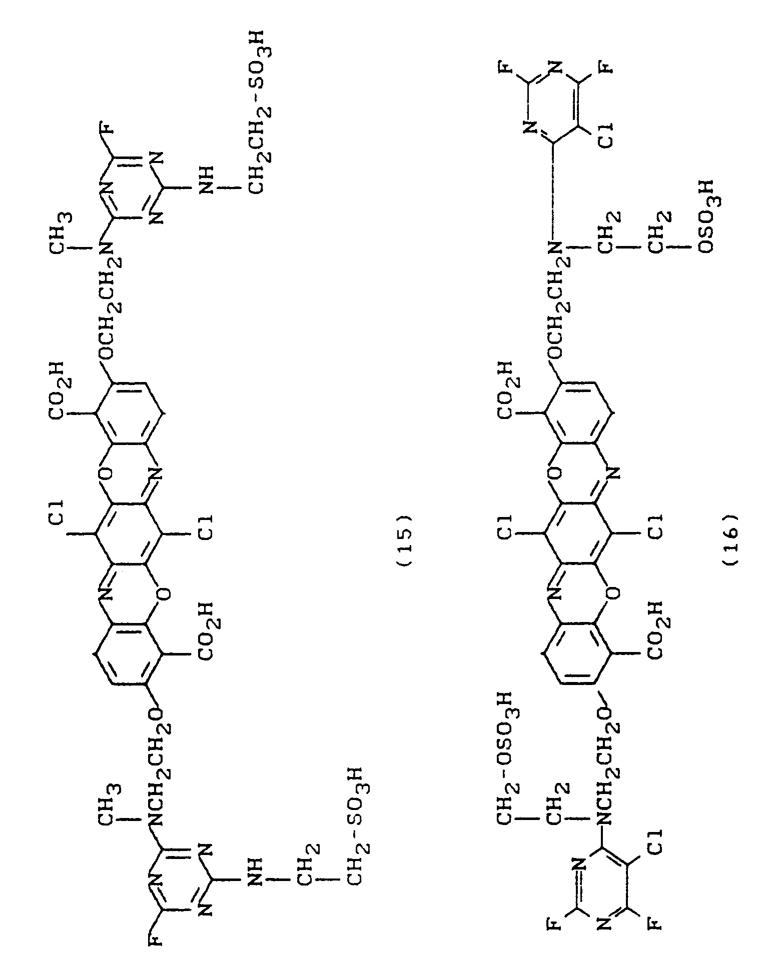
Farbstoff (12) mit Y = SO₃H sind auch darstellbar, indem man aktivierte 1,4-Benzochinone mit Aminoverbindungen (4) nach bekannten Methoden (z.B. Isopropanol/Natriumacetat (60 - 80°C)) zu dunkeloliven Farbstoffvorstufen der Struktur (17) kondensiert 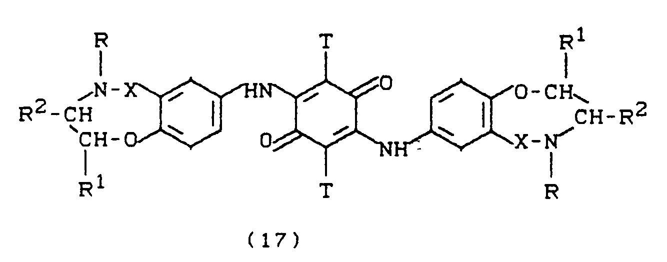
und diese anschließend in Oleum bei 60 - 120°C, vorzugsweise bei 75 - 85°C, unter gleichzeitiger Öffnung der Benzosultam- bzw. Benzolactam-Ringsysteme zu Triphendioxazinen cyclisiert. Dieses neue Verfahren ist ebenso wie die neuen roten Farbstoffe bzw. Farbstoffzwischenprodukte der Struktur (12) mit Y = SO₃H Gegenstand dieser Erfindung.

Kondensiert man (17) in Oleum bei etwa 20 - 40°C erhält man Farbstoffe der folgenden Struktur (18), 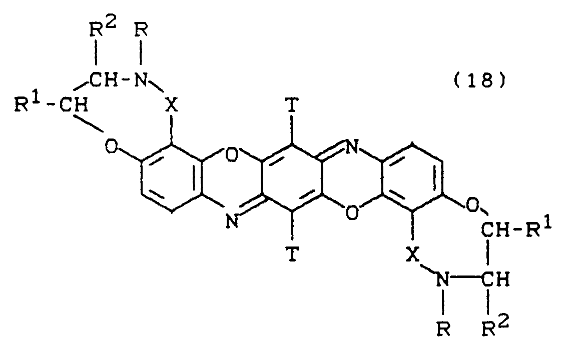
die ebenfalls Gegenstand der Erfindung sind.

Die Farbstoffe (12) mit Y - SO₃H eignen sich insbesondere zum Färben von Papier und Leder.

Farbstoffe (12) mit X = CO und Y = faserreaktive Gruppe sind neu und ebenfalls Gegenstand der Erfindung. Diese Farbstoffe eignen sich zum Färben von Baumwolle.

Farbstoffe (18) die wasserlöslich machende Gruppen aufweisen, eignen sich vorzugsweise zum Färben von Papier und Leder, solche die frei von wasserlöslich machenden Gruppen sind zum Färben von synthetischen Materialien bzw. Polyester.

### Beispiel 1:

244 g 8-Nitro-3,4-dihydro-(2H)-5,1,2-benzoxathiazin-1,1-dioxid, das durch intramolekulare Kondensation von 2-Chlor-5-nitro-N-(2-hydroxyethyl) benzolsulfonamid zugänglich ist (DOS 1 670 759), werden in 600 ml Methanol suspendiert und in Gegenwart von 3 g frisch bereitetem Raney-Nickel als Katalysator durch Aufdrücken der dreifachen äquimolaren Menge an Wasserstoff im Autoklaven bei 50°C reduziert. Nach vollständiger Reduktion wird der Katalysator von der heißen Reaktionslösung abgetrennt, aus der nach Abkühlen auf 0°C die Aminoverbindung in feinen Schuppen auskristallisiert. Nach Absaugen und Trocknen erhält man 205 g 8-Amino-3,4-dihydro-(2H)-5,1,2-benzoxathiazepin-1,1-dioxid der Formel 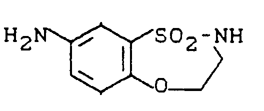
Schmp. 179°C
¹H-NMR(D₆-DMSO): δ = 3,36 (2H,t); 3,91 (2H,t); 5,30 (NH₂,s); 6,61 (1H,dd); 6,85 (1H,d); 6,94 (1H,d); 7,50 (NH,s) ppm.

In analoger Weise sind durch Reduktion folgende Aminoverbindungen (Bsp. 2,3 und 4) zugänglich:

### Beispiel 2:

### 8-Amino-2-methyl-3,4-dihydro-5,1,2-benzoxathiazepin-1,1-dioxid (Schmp. 148°C)

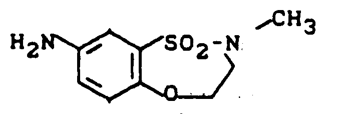

¹H-NMR (D₆-DMSO): δ = 2,62 (3H,s); 3,57 (2H,t); 3,97 (2H,t); 5,34 (NH₂,s); 6,65 (1H,dd); 6,84-6,88 (2H,m) ppm.

### Beispiel 3:

### 8-Amino-2-(2-hydroxyethyl)-3,4-dihydro-5,1,2-benzoxathiazepin-1,1-dioxid (Schmp. 141°C)

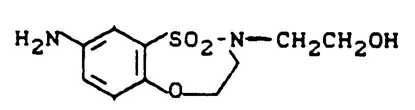

¹H-NMR (D₆-DMSO) : δ = 2,92 (2H,t); 3,53 (2H,m); 3,66 (2H,m); 3,99 (2H,m); 4,82 (OH,t); 5,36 (NH₂,s); 6,67 (1H,dd); 6,84 - 6,92 (2H,m) ppm.

### Beispiel 4:

### 8-Amino-2-phenyl-3,4-dihydro-5,1,2-benzoxathiazepin-1,1-dioxid (Schmp. 189°C)

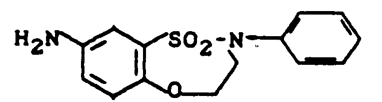

¹H-NMR (D₆-DMSO) : δ = 4,02 (2H,m); 4,10 (2H,m); 5,40 (NH₂,s); 6,74 (1H,dd); 6,89 (1H,d); 6,98 (1H,d); 7,11 (2H,d); 7,22 - 7,37 (3H,m) ppm.

Das zur Darstellung von Beispiel 3 benötigte 8-Nitro-2-(2-hydroxyethyl)-3,4-dihydro-5,1,2-benzoxathiazepin-1,1-dioxid ist nach folgenden 2 Synthesemethoden zugänglich:

### Weg A)

324,5 g N,N-Bis(2-hydroxyethyl)-2-chlor-5-nitrobenzolsulfonamid, das aus 2-Chlor-5-nitrobenzolsulfonylchlorid und Diethanolamin hergestellt wird, werden in 1 l Wasser mit 56 g festem Kaliumhydroxid unter Rückflußbedingungen erwärmt. Nach 1 Stunde Reaktionszeit kühlt man die Reaktionsmischung ab und isoliert das ausgefallene Produkt. Es werden 262 g einheitliches Nitrobenzosultam erhalten (Schmp. 124°C).

### Weg B)

122 g 8-Nitro-3,4-dihydro-(2H)-5,1,2-benzoxathiazepin-1,1-dioxid werden in 300 ml Wasser durch Zugabe von 20 g Natriumhydroxid gelöst. Man erwärmt die Lösung unter Rühren auf 60°C und leitet unter einer N₂-Schutzatmosphäre langsam ca. 30-35 g Ethylenoxid ein. Nach Zudosierung wird 2 Stunden nachgerührt und das überschüssige Ethylenoxid durch Stickstoffzufuhr entfernt. Die Reaktionsmischung wird neutralisiert und auf 20°C abgekühlt. Die Isolierung des kristallinen Niederschlages ergibt 129 g eines Alkylierungsproduktes, das sich mit dem nach Weg A isolierten 8-Nitro-2-(2-hydroxyethyl)-3,4-dihydro-5,1,2-benzoxathiazepin-1,1-dioxid als identisch erweist.

### Beispiel 5:

260 g N-(2-Hydroxyethyl)-N-methyl-2-chlor-5-nitrobenzoesäureamid, das aus 2-Chlor-5-nitrobenzoylchlorid und 2-(Methylamino)ethanol dargestellt wird, werden in 500 ml Wasser/500 ml Isopropanol auf Rückflußtemperatur erhitzt. Dazu tropft man innerhalb einer Stunde eine Lösung von 65 g Kaliumhydroxid in 250 ml Wasser und hält die Rückflußtemperatur für weitere 3 Stunden aufrecht. Die auf pH 9 abgestumpfte Reaktionslösung wird auf 0°C abgekühlt, der kristalline Niederschlag abgesaugt und getrocknet. Man erhält 174 g 7-Nitro-4-methyl-2,3-dihydro-1,4-benzoxazepin-5-on vom Schmp. 116°C. 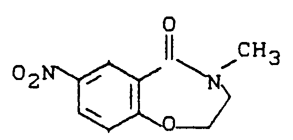
¹H-NMR (D₆-DMSO) : δ = 3,12 (3H,s); 3,71 (2H,m); 4,57 (2H,m); 7,17 (1H,d); 8,22 (1H,dd); 8,61 (1H,d)ppm.

### Beispiel 6:

144,5 g N,N-Bis(2-hydroxyethyl)-2-chlor-5-nitrobenzoesäureamid werden in 500 ml Wasser mit 28 g Kaliumhydroxid für 2 Stunden unter Rückflußkühlung erwärmt. Es wird auf 10°C abgekühlt, und das ausgefallene einheitliche Produkt abgesaugt und getrocknet. Es resultieren 84 g 7-Nitro-4-(2-hydroxyethyl)-2,3-dihydro-1,4-benzoxazepin-5-on vom Schmp. 151°C. 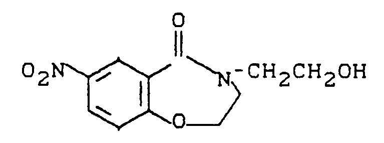
¹H-NMR (D₆-DMSO): δ = 3,67 (4H,m); 3,76 (2H,t); 4,58 (2H,t); 4,84 (OH,t); 7,18 (1H,d); 8,23 (1H,dd); 8,66 (1H,d) ppm.

### Beispiel 7:

111 g der unter Beispiel 5 erhaltenen Nitroverbindung werden in 450 ml Methanol suspendiert und in Gegenwart von 3 g frisch bereitetem Raney-Nickel als Katalysator durch Aufdrücken der dreifachen äquimolaren Menge an Wasserstoff im Autoklaven bei 50°C reduziert. Nach erfolgter Reduktion wird der Katalysator von der warmen Reaktionslösung abgetrennt. Die Lösung wird am Rotationsverdampfer aufkonzentriert. Man isoliert 91 g trockenes 7-Amino-4-methyl-2,3-dihydro-1,4-benzoxazepin-5-on vom Schmp. 165°C. 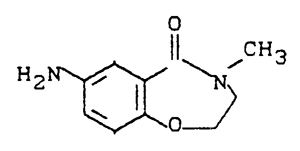
¹H-NMR (D₆-DMSO): δ = 3,04 (3H,s); 3,40 (2H,t); 4,13 (2H,t); 5,05 (NH₂,s); 6,61 (1H,dd); 6,68 (1H,d); 6,74 (1H,d)ppm.

### Beispiel 8:

Auf ähnliche Weise wie unter Pos. 7 wird die unter Beispiel 6 erhaltene Nitroverbindung zu 7-Amino-4-(2-hydroxyethyl)-2,3-dihydro-1,4-benzoxazepin-5-on vom Schmp. 138°C reduziert. 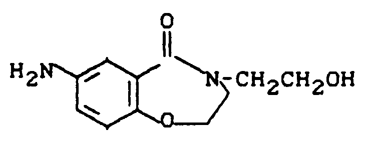
¹H-NMR (D₆-DMSO): δ = 3,44 (2H,t); 3,53 (4H,m); 4,13 (2H,t); 4,80 (OH,t); 4,99 (NH₂,s); 6,60 (1H,dd); 6,69 (1H,d); 6,72 (1H,d) ppm.

### Beispiel 9:

352 g (ca. 1,5 Mol) der nach Beispiel 1 erhaltenen Aminoverbindungen werden portionsweise in eine gerührte Vorlage aus 900 ml 20 %igem Oleum eingetragen. Die Temperatur sollte dabei 60°C nicht übersteigen. Anschließend erwärmt man unter Rühren für 2 Stunden auf 80°C. Es wird abkühlen lassen und auf eine Mischung aus 500 ml Wasser und insgesamt 3,5 kg Eis ausgetragen. Nachdem 10 - 15°C erreicht sind, wird die kristalline Fällung abgesaugt, mit 200 ml Wasser nachgewaschen und getrocknet. Es werden 537 g Amidosulfonsäure der Struktur (vermutlich Betainstruktur) 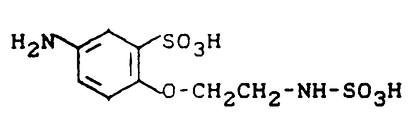
erhalten. Die Gehaltsbestimmung mittels Natriumnitritlösung liefert den Wert 38,5 g - 0,1 Mol, was einer Ausbeute von 93 % der Theorie entspricht.
¹H-NMR (D₂O,NaOD): δ = 3,36 (2H, t); 4,23 (2H,t); 6,91 (1H,dd); 6,98 (1H,d); 7,22 (1H,d) ppm.

| C,H,N-Analyse: | | | | | |
|---|---|---|---|---|---|
| ber.: | C 30,76 | H 3,85 | N 8,97 | O 35,9 | S 20,51 % |
| gef.: | C 30,0 | H 3,9 | N 8,7 | O 35,4 | S 20,1 % |

### Beispiel 10:

Verfährt man mit der Aminoverbindung aus Beispiel 2 analog wie unter Beispiel 9 aufgeführt, so resultiert eine substituierte N-(ß-Phenoxyethyl)amidosulfonsäure der Struktur 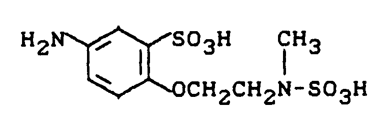
¹N-NMR (D₂O,NaOD): δ = 2,84 (3H,s); 3,41 (2H,t); 4,25 (2H,t); 6,95 (1H,dd); 7,03 (1H,d); 7,25 (1H,d) ppm.

### Beispiel 11:

300 g (1,2 Mol) des 8-Nitro-3,4-dihydro-(2H)-5,1,2-benzoxathiazepin-1,1-dioxids aus Beispiel 1, das nach DOS 1 670 759 zugänglich ist, werden portionsweise in eine gerührte Vorlage aus 800 ml 20 %igem Oleum eingetragen. Die Temperatur sollte dabei 60°C nicht übersteigen. Anschließend wird 1 Stunde auf 80 - 83 °C erwärmt. Da keine Ausgangsverbindung mehr chromatographisch nachweisbar ist, wird auf 20 °C abgekühlt und die Reaktionslösung auf eine Eis/Wasser-Mischung ausgetragen. Es werden portionsweise 48 g Natriumhydroxid eingetragen und 1 Stunde nachgerührt. Die kristalline Fällung wird isoliert und eine kleine Probe davon getrocknet. Nach NMR-Daten und Analyse kommt dem Hydrolyseprodukt die Struktur 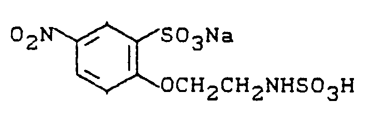
zu.
¹H-NMR (D₂O,NaOD): δ = 3,52 (2H,t); 4,48 (2H,t); 7,30 (1H,d); 8,35 (1H,dd); 8,57 (1H,d) ppm.

### Beispiel 12:

Den Rest der Isolierung, der feuchten Paste, aus Beispiel 11 rührt man in 800 ml Wasser an und stellt mit konz. Natronlauge unter Außenkühlung auf pH 8. Nach Zusatz von 5 g Raney-Nickel wird bei 25°C in Autoklaven Wasserstoff aufgedrückt. Für die Reduktion werden insgesamt 3,2 Mol Wasserstoff benötigt. Die Reaktionstemperatur steigt dabei auf 50°C an. Nach Beendigung der Wasserstoffaufnahme wird abgekühlt und die Apparatur entspannt. Durch Filtration trennt man den Katalysator von der Reaktionslösung ab. Diese wird mit 200 ml einer 48 %igen Schwefelsäure versetzt, wobei das Reduktionsprodukt langsam auskristallisiert. Nach Isolieren und Trocknen erhält man 340 g einer Aminoverbindung, die sich mit der Amidosulfonsäure des Beispiels 9 als identisch erweist. Der Gehalt an Aminoverbindung beträgt 95,8 % bzgl. einem Molgewicht von 334 (mono-Na-Salz), d.h. die Ausbeute über beide Reaktionsschritte entspricht 82 % d. Th..

### Beispiel 13:

250 g des Amidosulfonsäurederivats aus Beispiel 9 (81 %ig) werden in 2,2, l 10 %iger Salzsäure etwa 6 Stunden am Rückfluß gekocht, bis eine chromatographierte Probe kein Edukt mehr anzeigt. Nach Kaltrühren über Nacht fällt das Produkt der Formel 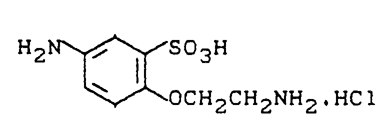
in Form von Nadeln aus, die abgesaugt und mit kalter 10 %iger Salzsäure abgedeckt und getrocknet werden. Zur analytischen Untersuchung wäscht man das Produkt besser mit Aceton salzsäurefrei und trocknet es. Man erhält 149,8 g des Hydrochlorids (86 % d. Th.), das sich mit einer auf anderem Wege synthetisierten Probe (nach DOS 3 635 312) als identisch erweist.
HCl ber. 13,59 % HCl gef. 13,63 %

### Beispiel 14:

46 g des Amino-benzolactams aus Beispiel 7 werden in 290 g 60 %iger Schwefelsäure für ca. 36 Stunden am Rückfluß erwärmt. Die Reaktionslösung wird abgekühlt und mit 250 ml Wasser verdünnt. Es werden langsam 160 g Calciumcarbonat eingetragen, bis ein pH-Wert von etwa 2 erreicht ist. Die Temperatur wird dabei durch Eiszugabe bei 20 - 25°C gehalten. Das ausgefallene Calciumsulfat wird abgetrennt, die resultierende Lösung mit verdünnter Natronlauge neutralisiert und bis zur beginnenden Kristallisation aufkonzentriert. Für die weitere Verwendung ist es jedoch günstiger, die Lösung der Verbindung der Formel 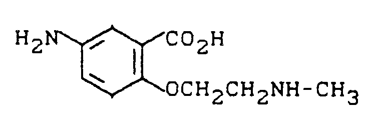
nach Neutralisation direkt mit entsprechenden Reaktionspartnern, wie z.B. Chloranil, umzusetzen.

### Beispiel 15:

21,8 g des Amino-benzolactams aus Beispiel 8 werden in 66 ml 20 %iger Salzsäure 8 Stunden bei 110°C am Rückfluß gekocht, bis das Edukt chromatographisch nicht mehr nachweisbar ist. Nach Abkühlen erhält man eine wasserklare Lösung, die nach Einengen im Vakuum plötzlich einen dicken Kristallbrei abscheidet, der nach Absaugen im Umlufttrockenschrank getrocknet werden kann. Es werden 30,3 g isoliert; das Produkt erweist sich nach argentometrischer Cl-Bestimmung als Bishydrochlorid der 5-Amino-2-[2-(2-hydroxyethylamino)ethoxy]benzoesäure der Struktur 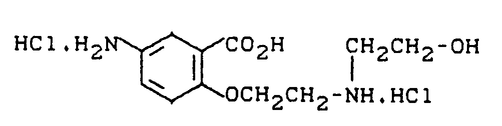
HCl ber. 23,3 % HCl gef. 23,4 %
¹H-NMR(D₆-DMSO): δ = 3,16 (2H,m); 3,37 (2H,m); 3,68 (2H,t); 4,41 (2H,t); 7,28 (1H,d); 7,55 (1H,dd); 9,13 (2H, breites s); 10,3 - 11,8 (ca. 2H) ppm.

### Beispiel 16:

44 g 8-Amino-3,4-dihydro-(2H)-5,1,2-benzoxathiazepin-1,1 dioxid (Bsp. 1), 25,1 g Chloranil (=2,3,5,6-Tetrachlor-1,4-benzochinon) und 31 g wasserfreies Natriumacetat werden 5 Stunden in 500 ml Isopropanol unter Rückflußkühlung erwärmt. Es wird abgekühlt und abgesaugt. Die braunolive Isolierung wird mit 50 ml Aceton und dreimal mit 200 ml Wasser gewaschen und dann getrocknet. Es resultieren 60,8 g des Biskondensationsproduktes der Struktur 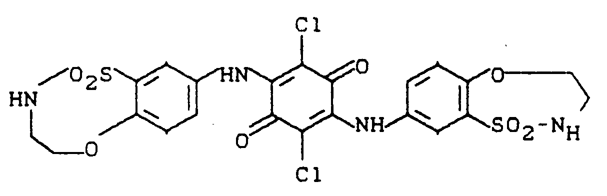
30 g dieses schwerlöslichen Biskondensationsproduktes werden portionsweise bei 30 - 40°C in 150 ml 20 %iges Oleum eingetragen. Es wird 3 Stunden unter Rühren auf 80- 85°C erwärmt. Die abgekühlte Reaktionslösung wird anschließend auf Eis ausgetragen, und die dunkelrote Ausfällung abgesaugt. Zur Neutralisation rührt man die isolierte Paste in 300 ml Wasser an und stellt mit verd. Natronlauge auf pH 6,5. Nach Zugabe von 30 g Kochsalz und 45 g Kaliumchlorid wird 1 Stunde gerührt und dann erneut durch Filtration isoliert. Nach dem Trocknen erhält man 26,1 g eines dunkelrotbraunen Farbstoffpulvers, dem die Struktur 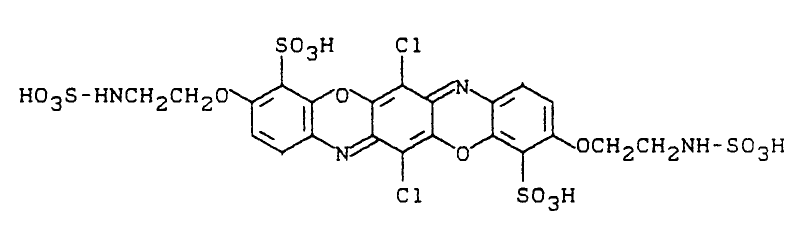
zukommt. Das neue Produkt kann z. B. als Ink-jet-Farbstoff eingesetzt werden und liefert dann auf Papier ein lichtechtes Magenta (λ max. = 540 nm (H₂O)).

## Patentansprüche

1. ß-Phenoxyethylamine der Struktur 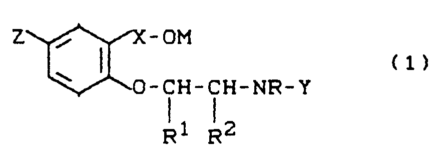 worin
Z = NH₂ oder NO₂
M = H, Alkali-, Erdalkali- oder Ammoniumkation
R = H oder ein gegebenenfalls substituierter (insbesondere durch OH, Cl, Br, CN, CO₂H, CO₂CH₃, CO₂C₂H₅, CONH₂, CON(CH₃)₂, SO₃H, OSO₃H sowie C₁-C₄-Alkoxy) C₁-C₄-Alkyl-, C₂-C₄-Alkenyl-oder C₂-C₄-Alkinyl-Rest oder
ein gegebenenfalls substituierter (insbesondere durch Halogen, C₁-C₄-Alkyl, CO₂H oder SO₃H) Phenyl-oder Benzyl-Rest,
R¹, R² = unabhängig voneinander H, C₁-C₄-Alkyl, Phenyl oder Benzyl, wobei R¹-R² auch Bestandteil eines cycloaliphatischen Restes sein können,
X = SO₂ oder CO und
Y = H oder SO₃M,
wobei
Y = SO₃M, wenn X = SO₂ und
Y = H, wenn X = CO.

2. Amine des Anspruchs 1 mit
R¹ und R² = H und R = C₁-C₄-Alkyl, CH₂CH₂OH, CH₂CO₂H oder CH₂CH₂OSO₃H.

3. Verfahren zur Herstellung der Phenoxyethylamine des Anspruchs 1 mit Z = NH₂, dadurch gekennzeichnet, daß man 2-Halogen-5-nitrobenzolsulfonsäureamide bzw. 2-Halogen-5-nitrobenzoesäureamide der Strukturen (2), 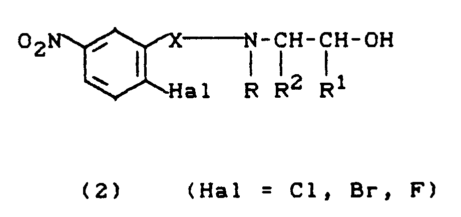 worin X, R, R¹ und R² die in Anspruch 1 genannte Bedeutung zukommt,
bei 80° bis 110°C in Gegenwart von Basen, insbesondere Alkalihydroxiden oder Alkalicarbonaten wie Natronlauge, Kalilauge, Soda, zu 5,1,2-Benzoxathiazepin-1,1-dioxiden (X = SO₂) bzw. 1,4-Benzoxazepin-5-onen (X=CO) der Formel 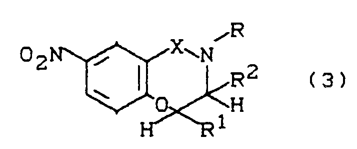 cyclisiert, diese Benzosultame bzw. Benzolactame anschließend katalytisch (z.B. Wasserstoff/Katalysator), mit Metall/Säure (z.B. Eisen/Essigsäure) oder mit anderen für aromatische Nitroverbindungen üblichen Reduktionsmitteln zu Aminoverbindungen der Struktur (4) reduziert 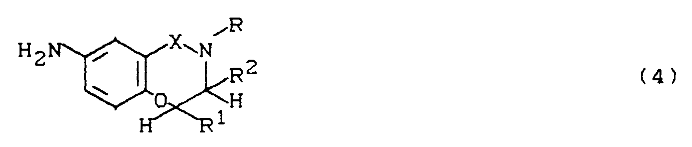 und diese abschließend unter Ringöffnung hydrolysiert.

4. Verfahren zur Herstellung der Phenoxyethylamine des Anspruchs 1, dadurch gekennzeichnet, daß man Benzosultame bzw. Benzolactame der Struktur 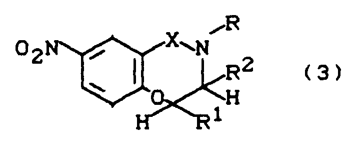 worin
R, R¹ und R² die in Anspruch 1 angegebene Bedeutung haben, zunächst zu Verbindungen der Formeln 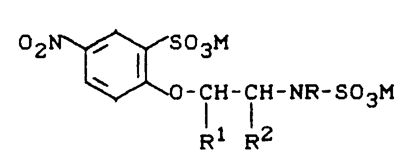 bzw. 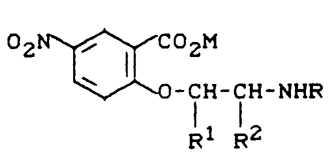 hydrolysiert und anschließend die Nitrogruppe zur Aminogruppe reduziert.

5. 7-Amino-1,4-benzoxazepin-5-one der Struktur 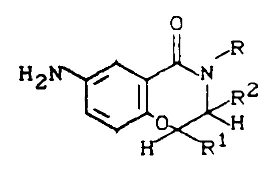 worin
R, R¹ und R² die in Anspruch 1 angegebene Bedeutung haben.

6. Verfahren zur Herstellung von Verbindungen der Struktur 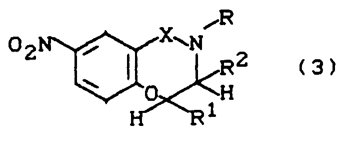 mit X = SO₂ und R = gegebenenfalls substituiertes C₁-C₄-Alkyl, C₂-C₄-Alkenyl oder C₂-C₄-Alkinyl, und
worin
R¹ und R² die in Anspruch 1 angegebene Bedeutung haben, dadurch gekennzeichnet, daß man Verbindungen (3) mit R=H unter Basenkatalyse mit entsprechenden Alkylierungsmitteln umsetzt.

7. Verfahren zur Herstellung von ß-Phenoxyethylaminen der Struktur 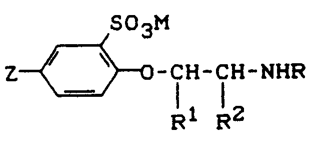 worin
Z = NO₂ oder NH₂
und R, R¹ und R² die in Anspruch 1 angegebene Bedeutung haben, dadurch gekennzeichnet, daß man Verbindungen an Struktur 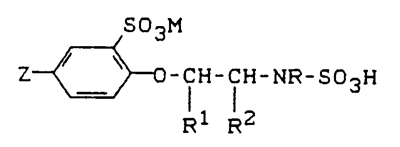 mit verdünnten Mineralsäuren hydrolysiert.

8. Verwendung der Verbindungen der Ansprüche 1, 2 und 5 zur Herstellung von Farbstoffen, insbesondere der Verbindungen mit Z = NH₂, zur Herstellung von Triphendioxazin-Farbstoffen oder Azofarbstoffen.

9. Farbstoffe der Formel 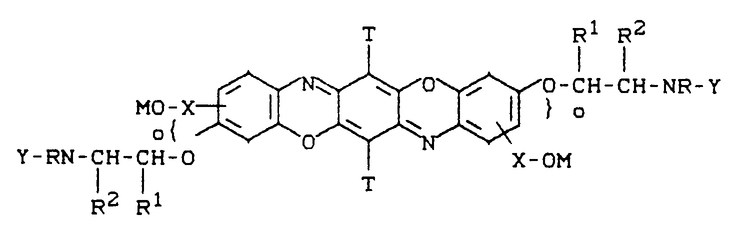 worin
T = Cl, Br, COOCH₃, OCH₃, 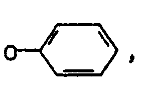
X = CO,
Y = faserreaktiver Rest und worin R, R¹, R² und M die in Anspruch 1 angegebene Bedeutung haben.

10. Farbstoffe der Formel 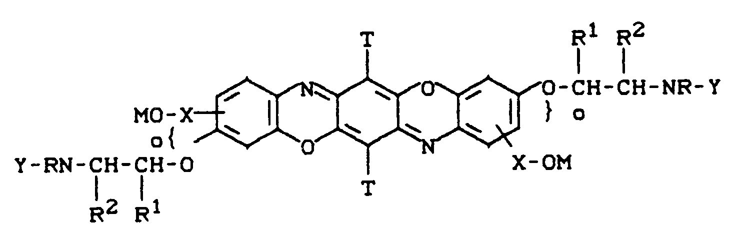 worin
T = Cl, Br, COOCH₃, OCH₃, 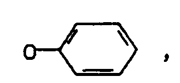
Y = SO₃H und wobei R, R¹, R² und M sowie X die in Anspruch 1 angegebene Bedeutung haben.

11. Farbstoffe der Formel 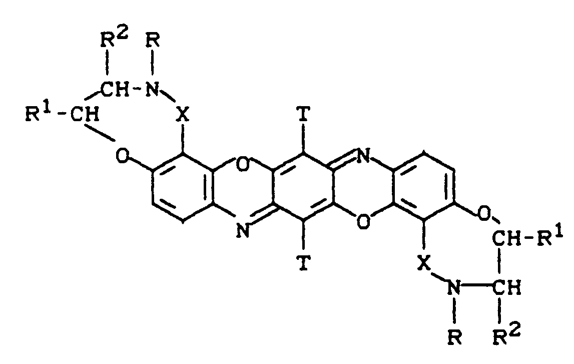 worin X, R, R¹, R² die in Anspruch 1 angegebene Bedeutung haben und T = Cl, Br, COOCH₃, OCH₃,

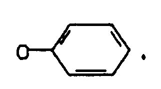

## Claims

1. β-Phenoxyethylamines of the structure 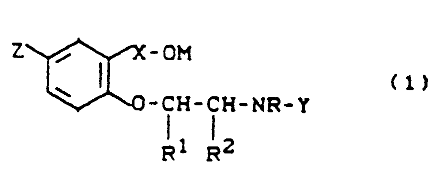 wherein
Z = NH₂ or NO₂,
M = H or an alkali metal cation, alkaline earth metal cation or ammonium cation,
R = H or a C₁-C₄-alkyl, C₂-C₄-alkenyl or C₂-C₄-alkinyl radical each of which is optionally substituted (in particular by OH, Cl, Br, CN, CO₂H, CO₂CH₃, CO₂C₂H₅, CONH₂, CON(CH₃)₂, SO₃H, OSO₃H and C₁-C₄-alkoxy) or
a phenyl or benzyl radical each of which is optionally substituted (in particular by halogen, C₁-C₄-alkyl, CO₂H or SO₃H),
R¹ and R² = independently of one another H, C₁-C₄-alkyl, phenyl or benzyl, it being also possible for R¹-R² to be a constituent of a cycloaliphatic radical,
X = SO₂ or CO and
Y = H or SO₃H,
Y being SO₃M if X = SO₂, and
Y being H if X = CO.

2. Amines of Claim 1 in which
R¹ and R² = H and R = C₁-C₄-alkyl, CH₂CH₂OH, CH₂CO₂H or CH₂CH₂OSO₃H.

3. Process for the preparation of the phenoxyethylamines of Claim 1 in which Z = NH₂, characterized in that 2-halogeno-5-nitrobenzenesulphonamides or 2-halogeno-5-nitrobenzamides of the structures (2) 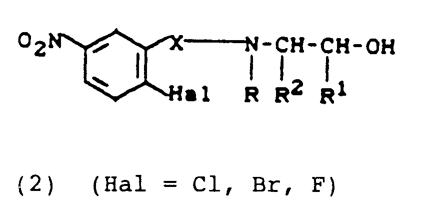 wherein
X, R, R¹ and R² have the meaning mentioned in Claim 1
are cyclized at 80° to 110°C in the presence of bases, in particular alkali metal hydroxides or alkali metal carbonates, such as sodium hydroxide solution, potassium hydroxide solution or sodium carbonate, to give 5,1,2-benzoxathiazepine 1,1-dioxides (X=SO₂) or 1,4-benzoxazepin-5-ones (X=CO), respectively, of the formula (3) 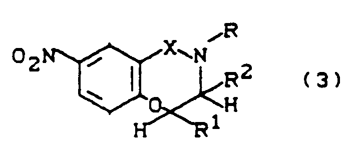 these benzosultams or benzolactams, respectively, are then reduced catalytically (for example hydrogen/catalyst), by means of metal/acid (for example iron/acetic acid) or by means of other reducing agents customary for aromatic nitro compounds to give amino compounds of the structure (4) 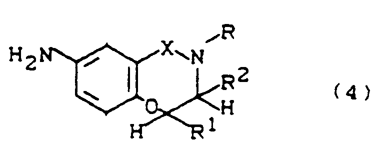 and the latter are finally hydrolyzed with ring opening.

4. Process for the preparation of the phenoxyethylamines of Claim 1, characterized in that benzosultams or benzolactams of the structure 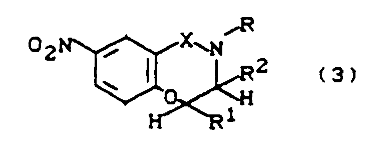 wherein
R, R¹ and R² have the meaning indicated in Claim 1 are first hydrolyzed to give compounds of the formulae 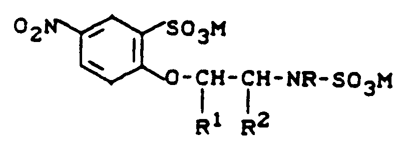 or 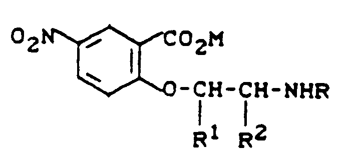 respectively, and the nitro group is then reduced to the amino group.

5. 7-Amino-1,4-benzoxazepin-5-ones of the structure 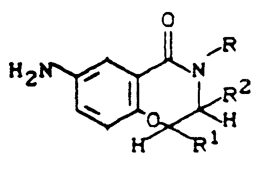 wherein
R, R¹ and R² have the meaning indicated in Claim 1.

6. Process for the preparation of compounds of the structure 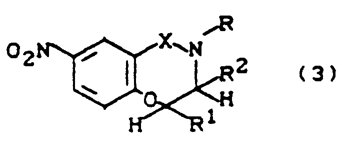 in which X = SO₂ and R = optionally substituted C₁-C₄-alkyl, C₂-C₄-alkenyl or C₂-C₄-alkinyl, and
wherein
R¹ and R² have the meaning indicated in Claim 1, characterized in that compounds (3) in which R=H are reacted with appropriate alkylating agents under base catalysis.

7. Process for the preparation of β-phenoxyethylamines of the structure 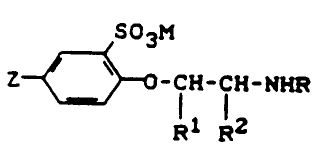 wherein
Z = NO₂ or NH₂
and R, R¹ and R² have the meaning indicated in Claim 1, characterized in that compounds of the structure 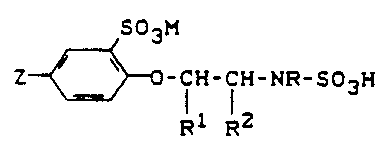 are hydrolyzed by means of dilute mineral acids.

8. The use of the compounds of Claims 1, 2 and 5 for the preparation of dyestuffs, in particular the compounds in which Z = NH₂, for the preparation of triphendioxazine dyestuffs or azo dyestuffs.

9. Dyestuffs of the formula 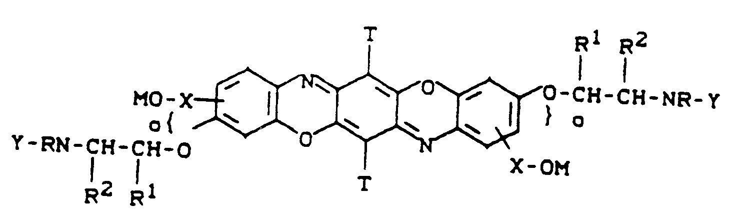 wherein
T = Cl, Br, COOCH₃, OCH₃ or 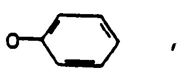
X = CO and
Y = a fibre-reactive radical and wherein R, R¹, R² and M have the meaning indicated in Claim 1.

10. Dyestuffs of the formula 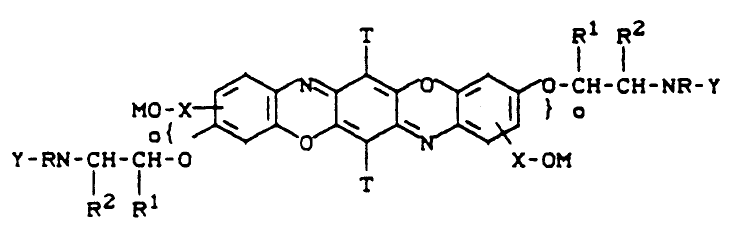 wherein
T = Cl, Br, COOCH₃, OCH₃ or 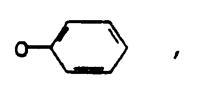
and Y = SO₃H and wherein R, R¹, R² and M and also X have the meaning indicated in Claim 1.

11. Dyestuffs of the formula 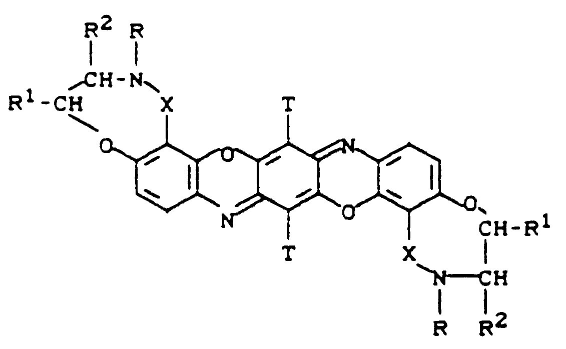 wherein X, R, R¹ and R² have the meaning indicated in Claim 1 and T = Cl, Br, COOCH₃, OCH₃ or

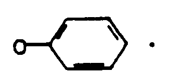

## Revendications

1. β-phénoxyéthylamines de formule 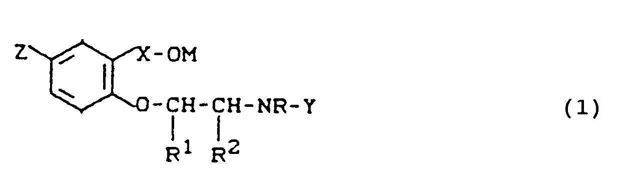 dans laquelle
Z = NH₂ ou NO₂
M = H, cation alcalin, alcalino-terreux ou ammonium
R = H ou un reste alkyle en C₁ à C₄, alcényle en C₂ à C₄ ou alcynyle en C₂ à C₄ éventuellement substitué (notamment par OH, Cl, Br, CN, CO₂H, CO₂CH₃, CO₂C₂H₅, CONH₂, CON(CH₃)₂, SO₃H, OSO₃H ainsi qu'alkoxy en C₁ à C₄), ou bien
un reste phényle ou benzyle éventuellement substltué (notament par un halogène, un radical alkyle en C₁ à C₄, CO₂H ou SO₃H),
R¹, R² = indépendamment l'un de l'autre, H, alkyle en C₁ à C₄, phényle ou benzyle, R¹-R² pouvant aussi être un constituant d'un reste cycloaliphatique
X = SO₂ ou CO et
Y = H ou SO₃M,
avec
Y = SO₃M lorsque X = SO₂ et
Y = H lorsque X = CO.

2. Amines suivant la revendication 1, dans lesquelles
R¹ et R² = H et R = alkyle en C₁ à C₄, CH₂CH₂OH, CH₂CO₂H ou CH₂CH₂OSO₃H.

3. Procédé de production des phénoxyéthylamines suivant la revendication 1 avec Z = NH₂, caractérisé en ce qu'on cyclise des 2-halogéno-5-nitrobenzènesulfonamides ou des 2-halogéno-5-nitrobenzamides de formule (2), 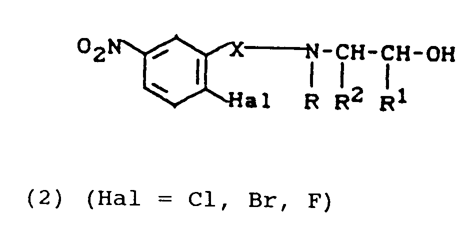 dans laquelle
X, R, R¹ et R² ont la définition indiquée dans la revendication 1,
à 80-110°C en présence de bases, notamment d'hydroxydes alcalins ou de carbonates alcalins tels que la lessive de soude, la lessive de potasse, le carbonate de sodium, en 1,1-dioxydes de 5,1,2-benzoxathiazépine (X = SO₂) ou en 1,4-benzoxazépine-5-ones (X = CO) de formule 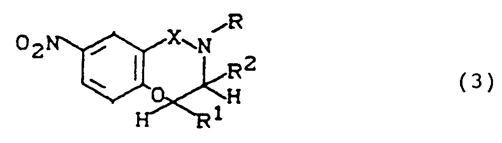 on réduit ensuite ces benzosultames ou benzolactames catalytiquement (par exemple hydrogène/catalyseur) avec un système métal/acide (par exemple fer/acide acétique) ou avec d'autres agents réducteurs d'emploi classique pour des composés aromatiques nitrés, en composés aminés de formule (4) 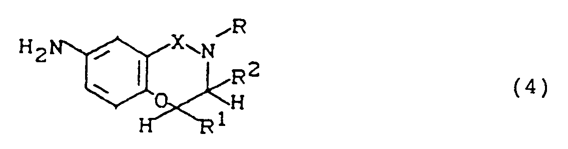 que l'on hydrolyse ensuite avec ouverture du noyau.

4. Procédé de production des phénoxyéthylamines suivant la revendication 1, caractérisé en ce qu'on hydrolyse tout d'abord des benzosultames ou benzolactames de formule 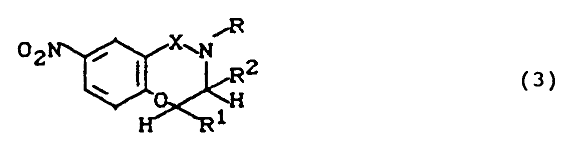 dans laquelle
R, R¹ et R² ont la définition indiquée dans la revendication 1, en composés de formules 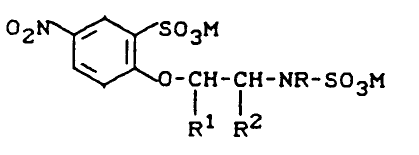 ou 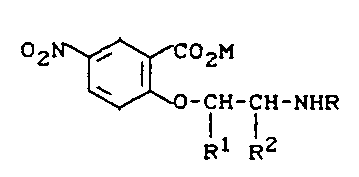
puis on réduit le groupe nitro en groupe amino.

5. 7-amino-1,4-benzoxazépine-5-ones de formule 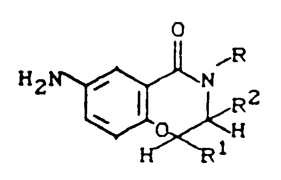 dans laquelle
R, R¹ et R² ont la définition indiquée dans la revendication 1.

6. Procédé de production de composés de formule 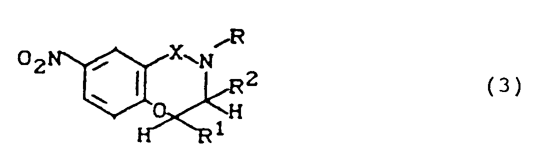 dans laquelle
X = SO₂ et R = groupe alkyle en C₁ à C₄, alcényle en C₂ à C₄ ou alcynyle en C₂ à C₄ éventuellement substitué, et
dans laquelle
R¹ et R² ont la définition indiquée dans la revendication 1, caractérisé en ce qu'on fait réagir des composés (3) dans lesquels R = H dans des conditions de catalyse basique, avec des agents alkylants correspondants.

7. Procédé de production de β-phénoxyéthylamines de formule 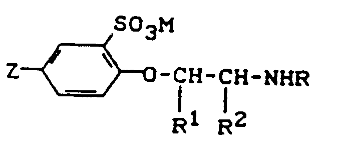 dans laquelle
Z = NO₂ ou NH₂
et R, R¹ et R² ont la définition indiquée dans la revendication 1, caractérisé en ce qu'on hydrolyse des composés de formule 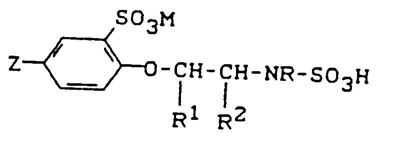 avec des acides minéraux dilués.

8. Utilisation des composés suivant les revendications 1, 2 et 5 pour la préparation de colorants, notamment des composés dans lesquels Z = NH₂ pour la préparation de colorants du type triphéndioxazine ou de colorants azoïques.

9. Colorants de formule 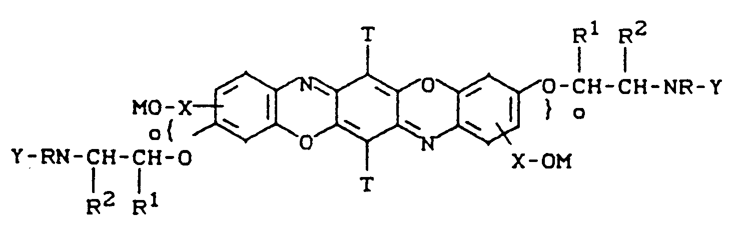 dans laquelle
T = Cl, Br, COOCH₃, OCH₃, 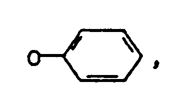
X = CO,
Y = reste réagissant avec la fibre, et R, R¹, R² et M ont la définition indiquée dans la revendication 1.

10. Colorants de formule 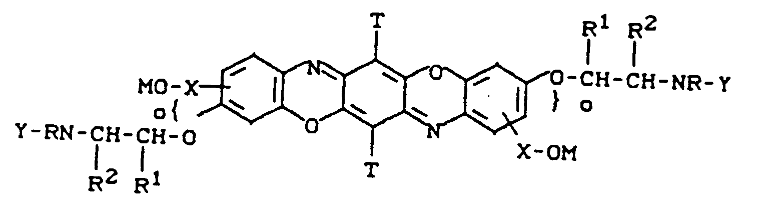 dans laquelle
T = Cl, Br, COOCH₃, OCH₃, 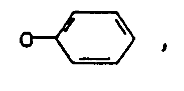
Y = SO₃H, et R, R¹, R² et M ainsi que X ont la définition indiquée dans la revendication 1.

11. Colorants de formule 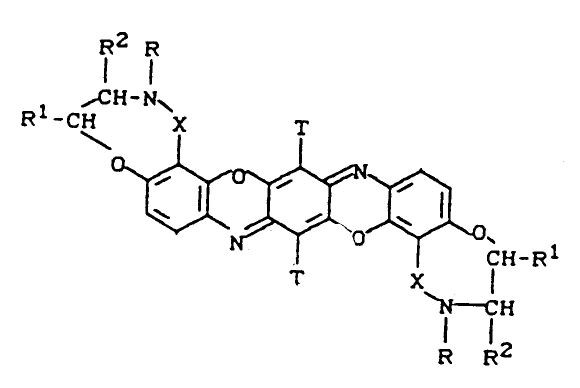 dans laquelle
X, R, R¹, R² ont la définition indiquée dans la revendication 1 et T = Cl, Br, COOCH₃, OCH₃,
